Europäisches Patentamt

European Patent Office

Office européen des brevets

(11) Publication number: **0 241 914 B1**

## EUROPEAN PATENT SPECIFICATION

(45) Date of publication of patent specification: **12.08.92**   (51) Int. Cl.5: **A61B 17/58**

(21) Application number: **87105516.6**

(22) Date of filing: **14.04.87**

(54) T-shaped plate for connecting bone splinters with bone shafts and associated screws.

(30) Priority: **14.04.86 PL 258954**

(43) Date of publication of application:
**21.10.87 Bulletin 87/43**

(45) Publication of the grant of the patent:
**12.08.92 Bulletin 92/33**

(84) Designated Contracting States:
**AT BE CH DE ES FR GB GR IT LI LU NL SE**

(56) References cited:
**EP-A- 0 077 681**
**CH-A- 462 375**
**US-A- 3 695 259**

**JOURNAL OF BONE AND JOINT SURGERY,
vol. 67-A, no. 4, April 1985, pages 550-555,
Boston, Massachusetts, US; P.E. SCRANTON
et al.: "Use of internal compression in arth-
rodesis of the ankle"**

(73) Proprietor: **Huta Baildon
ul. Zelazna 9
PL-40-952 Katowice(PL)**

(72) Inventor: **Karas, Wlodzimierz
ul. 27-go styczina 107/86
Dabrowa Gornicza(PL)**
Inventor: **Granowski, Robert, Dr. med.
ul.Swierczewskiego 71
Warszawa(PL)**
Inventor: **Ramotowski, Witold, Prof. Dr. med.
ul.Irysowa 106
Warszawa(PL)**
Inventor: **Tuziemski, Aleksander
ul. Gospodarcza 19/80
Sosnowiec(PL)**
Inventor: **Pilawski, Kazimierz
ul.Nowickiego 5/93
Warszawa(PL)**

(74) Representative: **Füchsle, Klaus, Dipl.-Ing. et al
Hoffmann . Eitle & Partner Patentanwälte Ar-
abellastrasse 4
W-8000 München 81(DE)**

Rank Xerox (UK) Business Services

## Description

This invention relates to a plate for connecting bone splinters with bone shafts.

Nowadays, in orthopeadic surgery there is a tendency to ensure, apart from connection of bone splinters, a firm internal stabilization thereof and to eliminate, in this way, a stiff dressing. This is a therapeutic method called stable osteosynthesis. One of variants of this therapy is the connection of bone splinters by means of plates with holes and bone screws.

Those skilled in the art know sets for a compressive connection of bones, consisting of a thrust plate screwed onto the bone by means of a screw with a cortex thread, with a head in the form of a shoulder, or a shaped screw nut, which is associated with a necessary operative cut indispensible for a repositioning of the fracture. In the case of some fracture of humeri, elbow osteosynthesis of humeri, fractures of upper tibia and some other lesions in the vicinity of the plate of connection, special T-shaped plates are used. A common feature of the known plates is a widened fixing part which has two, or more round holes. Hole axes are either parallel, or inclined at certain angle to one another. The fixing part is provided with several round holes, or a combination of round and oval holes.

During the first stage of the operation the fixing arms of the plates are fixed by means of screws to the bone splinters. Next, the shaft portion of the plate is fastened to the bone. If necessary, before fixing the shaft portion, the bone splinters are pressed axially to each other by means of a clamping device, or an automatic compression is used.

The design of the plate and screws so far used has not made possible stiffening of the fracture in the form of a stabilizer. In order to obtain a good stabilization of the splinters one must press the plate with a considerable force against the bone by means of screws. Such a pressure of the plate against the bone, together with isolation of a large bone area by the plate from the surrounding tissues either worsens, or completely precludes revascularization of the cortex layer lying under the plate. This has been confirmed by numerous histopathologic examinations of bone splinters taken from under the plate during removal of connecting material.

The article "Use of internal compression in arthrodesis of the ankle" in the Journal of Bone and Joint Surgery, Vol. 67-A, No. 4, April 1985, pages 550-555 by P.E. Scranton, describes a T-shaped plate for connecting bone splinters which has cylindrical holes in its shorter arm.

US-A-3695259 discloses a bone plate which comprises oval tapered holes and a groove in its bottom portion extending in the major direction of the plate.

The object of the invention is to provide a plate which avoids a lesion of the bone fracture being treated, or avoids disturbances making impossible the correct course of treatment.

The object of the invention is solved by providing a T-shaped plate for connecting bone splinters, and bone screws having a cortex thread on one end, a screw thread on the other end and a shoulder located therebetween, the transverse and longitudinal arms of said plate being provided with oval tapered pressure holes, the longitudinal axis of said oval tapered pressure holes being parallel to the longitudinal axis of the longitudinal arm, the middle point of the oval form of the small diameter part of the oval tapered pressure holes in the transverse arm being offset from the middle point of the larger diameter part of said oval tapered pressure holes in a direction away from the longitudinal arm; the middle point of the oval form of the small diameter part of the further oval tapered pressure holes in the longitudinal arm being offset from the middle of the larger diameter part of said further oval tapered pressure holes in a direction away from the transverse arm, and said transverse and longitudinal arms have grooves in their bottom portion in the same axis in which the oval tapered pressure holes are situated and are formed in such a way as to receive the shoulder of the respective bone screws when engaged into the holes.

The plate according to the invention is used with bone screws with cortex thread on one side and metric thread on the other side. The plate is superimposed on the screws which have been previously screwed into the bone splinters and screwed thereto with nuts put on the side of the metric thread is a structure having the features of a clamp stabilizer. The plate makes possible connection of fractures of anatomical neck of humeri, elbow bases of humeri, upper tibia bases, as well as other lesions in the neighbourhood of base. Owing to its design, the application of a double thread separated from each other by a suitable shoulder, the plate may be used either under skin, or above skin. Elevation of the plate above the bone eliminates its pressure against the bone and isolation by the plate of a large area of the bone from the surrounding tissues which prevents revascularization of the cortex layer situated under the plate.

This invention can be more fully understood from the following detailed description when taken in conjunction with the accompanying drawings, wherein

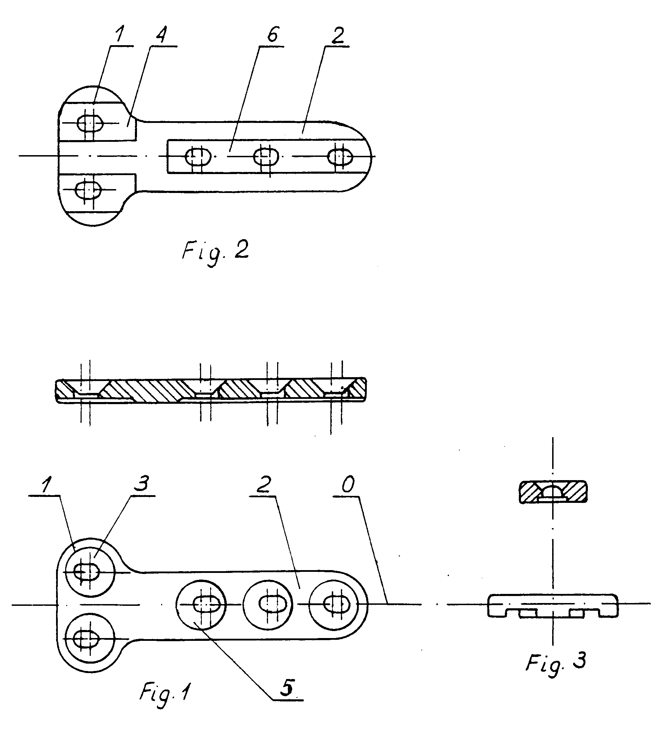

Fig. 1 presents the top view of the plate,
Fig. 2 visualizes the bottom view of the plate
Fig. 3 presents the side view of the plate when viewed upon from the side of the base part,

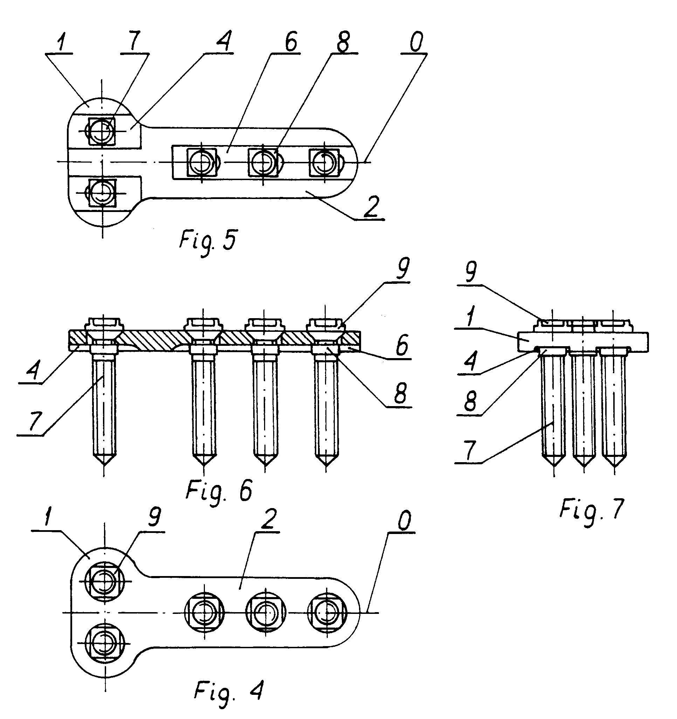

Fig. 4 shows the top view of the plate, together with screws,

Fig.5 shows the bottom view of the plate, together with screws,

Fig. 6 presents the longitudinal section of the plate, together with screws,

Fig. 7 visualizes side view of the plate viewed upon from the side of the base part, together with screws.

The plate has a shorter base arm 1 and a longer shaft arm 2 situated perpendicularly to each other. In the base arm 1 there are two oval tapered pressure holes 3, of the same direction as the longitudinal axis 0 of the longer shaft arm 2. In the bottom portion of the base arm 1 there are grooves 4, lying in the same axis, where all oval tapered pressure holes 3 are situated. In the longer shaft arm 2 in the longitudinal axis 0 there are oval tapered pressure holes 5. In the bottom portion of the shaft arm 2 in its longitudinal axis 0 there runs a groove 6.

The plate according to the invention is to be mounted in the following way. At first, screws 7 with cortex thread on one end and metric thread on the other, the side with cortex thread is screwed into the holes drilled in the broken bone so that the shoulder 8 be not in contact with the bone. Next, on the screws 7 thus screwed a plate is to be introduced so that the shorter base arm 1 rests on the screws 7, introduced into the base part of the bone, and the longer shaft arm 2 rests on the screws 7, introduced into the bone shaft. Shoulders 8 of screws 7 enter the grooves 4 and 6. On the portions of the screws 7 with metric thread protruding above the oval tapered holes 3 and 5 suitable nuts 9 should be screwed down. Tightening of nuts 9 is accompanied by a displacement of the screw 7 with oval tapered hole 3 and 5. When the screws 7 are being shifted the fracture fissure is being closed and pressed.

## Claims

1. A T-shaped plate for connecting bone splinters, and bone screws having a cortex thread on one end, a screw thread on the other end and a shoulder (8) located therebetween,

   the transverse arm (1) and the longitudinal arm (2) of said T-shaped plate being provided with oval tapered pressure holes (3, 5), the longitudinal axis of said oval tapered pressure holes (3, 5) being parallel to the longitudinal axis (0) of the longitudinal arm (2);

   the middle point of the oval form of the small diameter part of the oval tapered pressure holes (3) in the transverse arm (1) being offset from the middle point of the larger diameter part of said oval tapered pressure holes (3) in a direction away from the longitudinal arm (2);

   the middle point of the oval form of the small diameter part of the further oval tapered pressure holes (5) in the longitudinal arm (2) being offset from the middle of the larger diameter part of said further oval tapered pressure holes (5) in a direction away from the transverse arm (1), and said transverse arm (1) and longitudinal arm (2) have longitudinal grooves (4, 6) in their bottom portion in the same axis in which the oval tapered pressure holes (3, 5) are situated and are formed in such a way as to receive the shoulder (8) of the respective bone screws when engaged into the holes.

## Revendications

1. Plaque en forme de T pour relier des esquilles d'os, et des vis pour os ayant un filetage côté écorce à une extrémité, un filetage de vis à l'autre extrémité et un épaulement (8) située entre eux,

   - la branche transversale (1) et la branche longitudinale (2) de ladite plaque en forme de T étant munies de trous presseurs coniques ovales (3, 5), l'axe longitudinal desdits trous presseurs coniques ovales (3, 5) étant parallèle à l'axe longitudinal (0) de la branche longitudinale (2); le point médian de la forme ovale de la partie à petit diamètre des trous presseurs coniques ovales (3) dans la branche transversale (1) étant décalé par rapport au point médian de la partie à grand diamètre desdits trous presseurs coniques ovales (3) dans une direction orientée en sens opposé à la branche longitudinale (2);

   - le point médian de la forme ovale de la partie à petit diamètre des autres trous presseurs coniques ovales (5) dans la branche longitudinale (2) étant décalé par rapport au milieu de la partie à grand diamètro desdits autres trous presseurs coniques ovales (5) dans une direction orientée en sens opposé à la branche transversale (1), et lesdites branche transversale (1) et branche longitudinale (2) présentent des rainures longitudinales (4, 6) dans leur partie inférieure suivant le même axe dans lequel les trous presseurs coniques ovales (3, 5) sont situés, et sont formées de façon à recevoir l'épaulement (8) des vis respectives des os lorsqu'elles sont engagées dans les trous.

**Patentansprüche**

1.  Eine T-förmige Platte zur Verbindung von Knochenbruchstücken und Knochenschrauben mit einem Cortexgewinde an einem Ende, einem Schraubengewinde an dem anderen Ende und einer sich dazwischen befindlichen Schulter (8),
    wobei der Querarm (1) und der Längsarm (2) der T-förmigen Platte mit ovalen sich verjüngenden Drucklöchern (3, 5) versehen ist, wobei die Längsachse der ovalen sich verjüngenden Drucklöchern (3, 5) parallel zu der Längsachse (0) des Längsarmes (2) ist;
    wobei der Mittelpunkt der ovalen Form des kleineren Durchmesserteils der sich oval verjüngenden Drucklocher (3) in dem Querarm (1) vom Mittelpunkt des größeren Durchmesserteils der ovalen sich verjüngenden Drucklöcher (3) in einer Richtung weg von dem Längsarm (2) versetzt sind;
    wobei der Mittelpunkt der ovalen Form des kleineren Durchmesserteils der weiteren ovalen sich verjüngenden Drucklöchern (5) in dem Längsarm (2) von der Mitte des größeren Durchmesserteils der weiteren sich oval verjüngenden Drucklöcher (5) in einer Richtung weg von dem Querarm (1) versetzt sind, und der Querarm (1) und Längsarm (2) Längsausnehmungen (4, 6) in ihren Bodenabschnitt in derselben Achse aufweisen, in der sich die ovalen sich verjüngenden Drucklöcher (3, 5) befinden und derart geformt sind, um die Schulter (8) der jeweiligen Knochenschrauben bei Eingriff in die Löcher zu empfangen.

Fig. 2

Fig. 1

Fig. 3

Fig. 5

Fig. 6

Fig. 7

Fig. 4